# EUROPÄISCHE PATENTSCHRIFT

(11) **EP 1 405 607 B1**
(45) Veröffentlichungstag und Bekanntmachung des Hinweises auf die Patenterteilung: **16.10.2013**
(21) Anmeldenummer: 03019180.3
(22) Anmeldetag: 25.08.2003
(51) Int. Cl.: A61B 17/86

(54) **Knochenschraube mit Halteelement**
Bone screw with holding element
Vis à os avec élement de maintien

(30) Priorität: 04.10.2002 DE 10246386
(43) Veröffentlichungstag der Anmeldung: 07.04.2004
(60) Teilanmeldung: 07014106.4
(73) Patentinhaber: Biedermann Technologies GmbH & Co. KG, 78166 Donaueschingen (DE)
(72) Erfinder: Biedermann, Lutz, 78048 VS-Villingen (DE); Harms, Jürgen, 76227 Karlsruhe (DE)
(74) Vertreter: Hofer, Dorothea

(56) Entgegenhaltungen:
- EP-A2- 1 222 899
- WO-A-02/38054
- FR-A1- 2 820 630
- JP-A- 2001 017 440
- US-A- 5 645 546
- US-A- 5 964 761
- US-A- 6 048 343
- US-A1- 2002 055 783

## Beschreibung

Die vorliegende Erfindung bezieht sich auf eine Knochenschraube, insbesondere auf eine Knochenschraube mit einem Gewindeabschnitt mit einem netzartigen Aufbau.

Eine derartige Knochenschraube ist aus der DE 100 55 891 A1 bekannt. Sie beinhaltet einen Gewindeabschnitt, eine Spitze an dem einen Ende und einen Kopf zum Eingreifen mit einem Schraubendreher an dem anderen Ende. Der Gewindeabschnitt ist rohrförmig ausgebildet und seine Wandung weist eine Mehrzahl von Ausnehmungen auf.

Die bekannte Schraube kann z.B. bei der Behandlung von osteoporotischen Frakturen eingesetzt werden. Aufgrund der netzartigen Ausbildung der Schraube kann eine Fusion mit umgebendem Knochenmaterial stattfinden. Die Position der Fraktur im Knochen, die z.B. beim Hüftknochen oftmals weit von der Oberfläche entfernt ist, von der aus die Schraube eingeschraubt wird, erfordert zur sicheren Fixation eine präzise Positionierung der Knochenschraube und zum Teil auch eine mechanische Verbindung zu externen und/oder internen Verstrebungsimplantaten verbunden werden kann.

Aus der US 6,048,343 ist ein Knochenschraubensystem bekannt, das eine Knochenschraube mit einem Kanal aufweist und einen Adapter, der lösbar mit der Schraube verbunden ist. Die Knochenschraube hat einen Kopf mit einer Öffnung, in die der Adapter einführbar ist, sowie ein geschlossenes Ende und Öffnungen im Schaft.

Der vorliegenden Erfindung liegt die Aufgabe zugrunde, eine Knochenschraube der oben beschriebenen Art und eine Knochenfixationseinrichtung mit verbesserten Eigenschaften und Einsatzmöglichkeiten bereitzustellen, die insbesondere präzise positionierbar ist und wahlweise auch mit externen und/oder internen Verstrebungsimplantaten.

Die Aufgabe wird gelöst durch eine Knochenschraube gemäß Anspruch 1, und eine Knochenfixationseinrichtung gemäß Anspruch 13 bzw. 14 . Weiterbildungen der Erfindung sind in den Unteransprüchen gekennzeichnet.

Die erfindungsgemäße Knochenschraube weist insbesondere den Vorteil auf, dass sie im Knochen an die erforderliche Stelle versenkbar ist. Somit erfüllt sie ihre Funktion als Zugelement, ohne dass ein Teil aus dem Knochen heraussteht.

Ferner lassen sich Knochenzement oder Wirkstoffe mittels der Knochenschraube präzise platzieren.

Weitere Merkmale und Zweckmäßigkeiten der Erfindung ergeben sich aus der Beschreibung von Ausführungsbeispielen anhand der beigefügten Zeichnungen. Von den Figuren zeigen:
- Fig. 1: eine Explosionsdarstellung des modularen Aufbaus einer Knochenschraube gemäß einer ersten bis dritten Ausführungsform der vorliegenden Erfindung;
- Fig. 2: eine Darstellung der in Fig. 1 gezeigten Knochenschraube in zusammengesetztem Zustand;
- Fig. 3: eine Schrittfolge zum Befestigen einer äußeren Platte an einem Knochen.
- Fig. 4: eine Darstellung einer Kombination der Knochenschraube mit einem Marknagel.
- Fig. 5: eine Darstellung einer Knochenschraube gemäß einer vierten Ausführungsform der vorliegenden Erfindung;

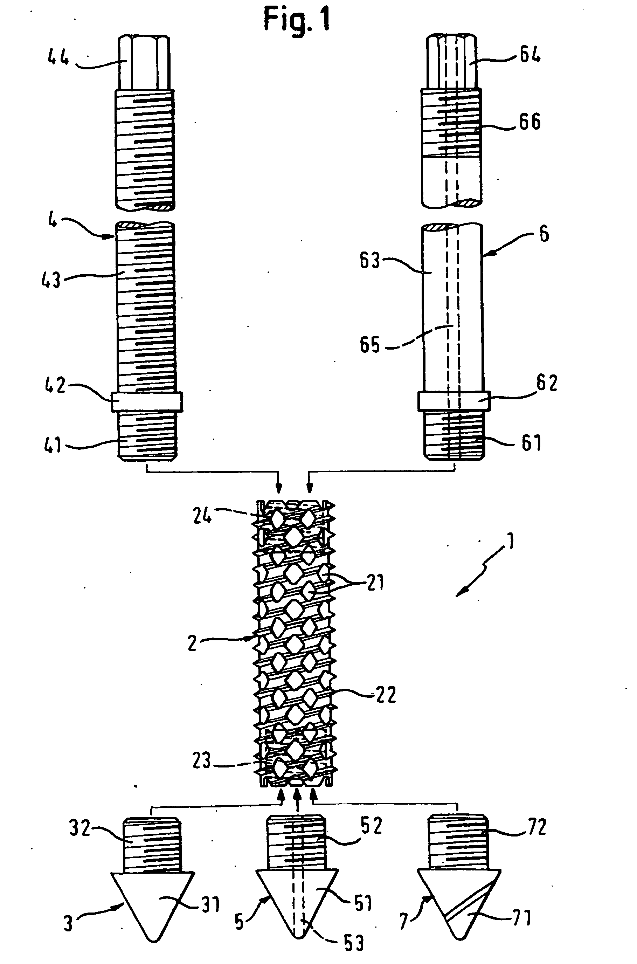

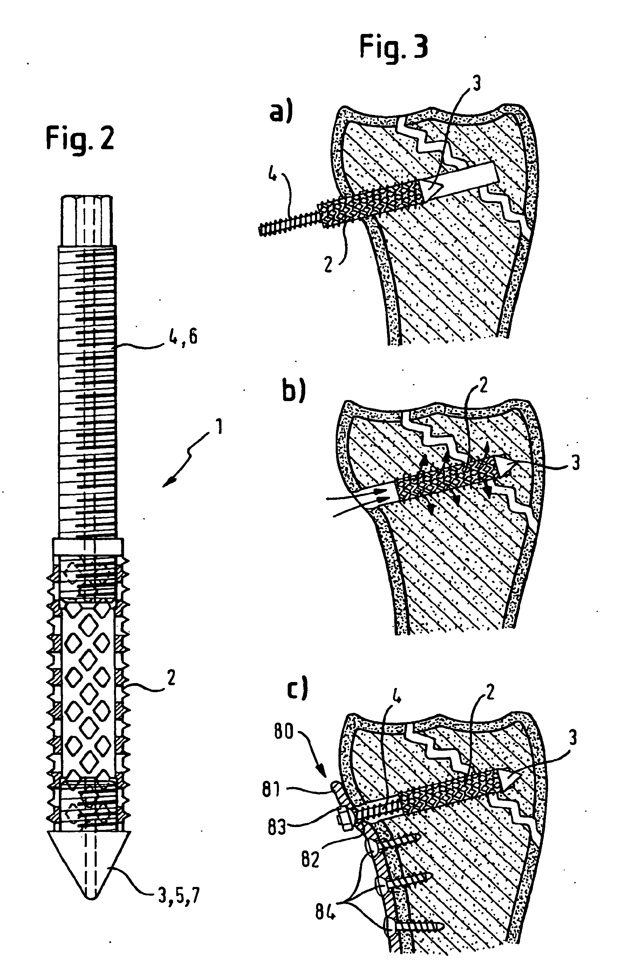

Im folgenden wird mit Bezug auf Fig. 1 und 2 eine Knochenschraube nach einer Ausführungsform der vorliegenden Erfindung beschrieben. Dabei ist in Fig. 2 zur Verbesserung der Übersichtlichkeit ein Teil der Knochenschraube im Schnitt dargestellt.

Eine erste Ausführungsform der in Fig. 1 und 2 dargestellten modularen Knochenschraube 1 beinhaltet einen Gewindeabschnitt 2, eine Spitze 3 und ein Halteelement 4.

Der Gewindeabschnitt 2 ist rohrförmig ausgebildet und weist in seiner Wandung eine Vielzahl von Ausnehmungen 21 auf, die in dem gezeigten Ausführungsbeispiel rautenförmig ausgebildet sind. Die Rauten sind derart ausgerichtet, dass sich ihre Symmetrieachse jeweils parallel zu der Symmetrieachse des Rohres erstreckt. Die Rauten sind ferner gegeneinander versetzt angeordnet.

Auf der Außenwandung ist ein sogenanntes Knochengewinde 22 vorgesehen, welches in seiner Form den üblichen Knochen-schrauben entspricht.

Auf der Innenwandung weist der rohrförmige Gewindeabschnitt 2 an seinen beiden Enden je ein metrisches Innengewinde 23, 24 auf .

Die Spitze 3 beinhaltet den eigentlichen Spitzenteil 31 und einen Schaft 32. In dem gezeigten Ausführungsbeispiel weist der Schaft 32 ein Außengewinde auf, das dem Innengewinde 23 des rohrförmigen Gewindeabschnitts 2 entspricht, zum Verbinden der Spitze 3 mit dem Gewindeabschnitt 2 wie in Fig. 2 dargestellt.

Das Halteelement 4 weist an einem ersten Ende einen ersten Abschnitt 41 mit einem Außengewinde auf, das dem Innengewinde 23 des rohrförmigen Gewindeabschnitts 2 entspricht, zum Verbin-den des Halteelements 4 mit dem Gewindeabschnitt 2 wie in Fig. 2 dargestellt.

An dem Ende des Gewindeabschnitts 41 ist ein Anschlag 42 in Form einer Schulter bzw. eines ringförmigen Vorsprungs vorgesehen. Durch diesen Anschlag wird der Einschraubweg beim Einschrauben begrenzt. Wie insbesondere aus Fig. 2 ersichtlich, entspricht der Außendurchmesser des ringförmigen Vorsprungs 42 dem Außendurchmesser des rohrförmigen Gewindeabschnitts 2, so dass er beim Einschrauben der Knochenschraube in der durch diese geschaffenen Öffnung kein Hindernis darstellt.

Das Halteelement weist weiterhin einen stabförmigen Abschnitt 43 auf, der wie in Fig. 2 dargestellt in eingeschraubtem Zustand über das zweite Ende des Gewindeabschnitts 2 hinausragt. Der stabförmige Abschnitt 43 ist vorzugsweise durchgehend mit einem metrischen Außengewinde versehen.

Die axiale Länge des stabförmigen zweiten Abschnitts 43 ist bevorzugt größer als die des ersten, in den Gewindeabschnitt 2 einzuschraubenden Abschnitts 41. Sie ist in Abhängigkeit von dem Einsatzort der Knochenschraube so gewählt, dass der rohrförmige Gewindeabschnitt 2 an die erforderliche Stelle in dem Knochen versenkbar ist.

An dem seinem dem Abschnitt 41 gegenüberliegenden freien Ende weist das Halteelement ein Angriffselement für ein Einschraubwerkzeug in Form eines Außensechskantabschnitts 44 auf.

Der Gewindeabschnitt 2, die Spitze 3 und das Halteelement 4 sind vorzugsweise aus Titan geformt. Es kann jedoch auch ein anderes biokompatibles Material verwendet werden.

Im folgenden wird mit Bezug auf Fig. 2 und 3 der Einsatz der Knochenschraube nach der ersten Ausführungsform beschrieben. Die Knochenschraube 1 wird dabei z.B. in das Ende eines Röhrenknochens eingesetzt, der durch Osteoporose von innen her geschwächt ist, z.B. in den Schenkelhals eines Oberschenkelknochens.

Zunächst wird die Spitze 3 in den rohrförmigen Gewindeabschnitt 2 eingeschraubt. Als nächstes wird das Halteelement 4 in den Gewindeabschnitt 2 eingeschraubt. Dadurch erhält man den in Fig. 2 dargestellten Aufbau.

Wie in Fig. 3 a) dargestellt, wird als nächstes die aus Gewindeabschnitt 2, Spitze 3 und Halteelement 4 zusammen-gesetzte Knochenschraube 1 in den Knochen hinein geschraubt. Dabei kann der Gewindeabschnitt 2 tief in den Knochen versenkt und dabei präzise an der Frakturstelle positioniert werden. Anschließend wird das Halteelement 4 wieder aus dem Gewindeabschnitt 2 herausgeschraubt.

Wie in Fig. 3 b) dargestellt, wird in einem weiteren Schritt in das Innere des Gewindeabschnitts 2 ein Füllmaterial wie z.B. Knochenzement oder Ähnliches und/oder ein medikamentöser oder wachstumsfördernder Wirkstoff eingespritzt. Im Gegensatz zu dem direkten Einspritzen sorgt der Gewindeabschnitt 2 für eine genauere Positionierung des Einspritzkanals und für eine gleichmäßigere Verteilung des eingespritzten Materials, das durch die Ausnehmungen 21 in die benachbarten Abschnitte des Knochens austreten kann. Dadurch wird die Fixierung des Knochens durch die Schraube weiter verbessert.

Die versenkte Knochenschraube dient als Verstärkung für die z.B. durch Osteoporose geschwächten Knochenbälkchen. Durch die Ausnehmungen 21 kann der Knochen in die Schraube einwachsen. Eine Stabilisierung der Frakturstelle des geschwächten Knochens erfolgt somit als Kombination von Zugentlastung und Fusion.

Fig. 3 c) zeigt eine Fixationseinrichtung 80 zum Fixieren des Knochens mit Hilfe einer äußeren Fixationsplatte. Dabei wird das Halteelement 4 nach dem Einspritzen des Füllmaterials wieder in den Gewindeabschnitt 2 eingeschraubt. Eine Platte 81 mit einer Ausnehmung 82 wird so an den Knochen angelegt, dass das freie Ende des Halteelements 4 durch die Ausnehmung 82 ragt, und durch Aufschrauben einer Mutter 83 auf das Außenge-winde des stabförmigen Abschnitts 43 des Halteelements 4 fest mit der Knochenschraube 1 verbunden. Weitere Knochenschrauben 84 dienen zum stabilen Fixieren der Platte 81 an dem Knochen. Der stabförmige Abschnitt 43 kann hierzu auf die erforderliche Länge gekürzt werden, die so bemessen ist, dass er nicht wesentlich über die Mutter 83 hervorsteht.

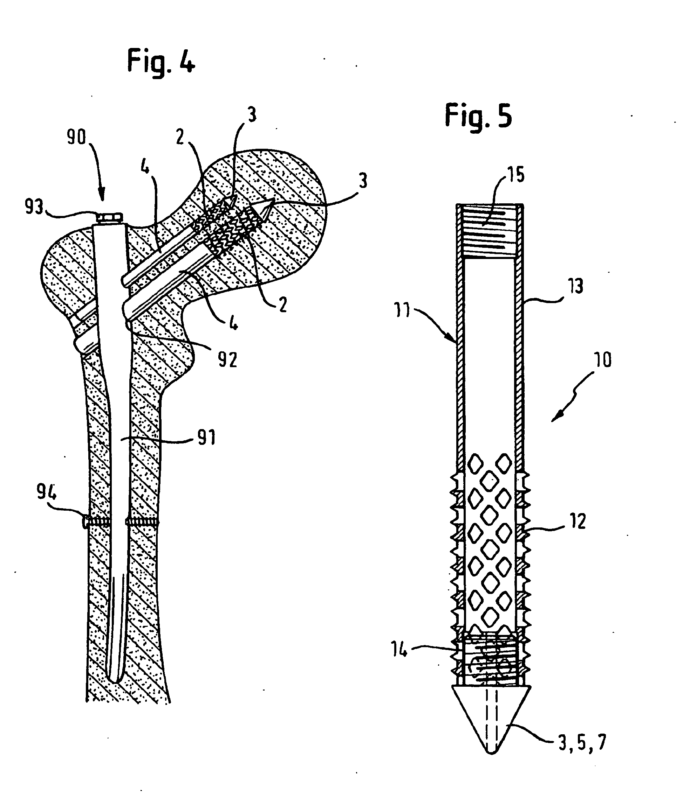

Fig. 4 zeigt ein weiteres Anwendungsbeispiel für die Knochenschraube nach der ersten Ausführungsform. Bei der Fixationseinrichtung 90 erfolgt die Fixierung des Knochens nicht Hilfe einer äußeren Fixationsplatte, sondern mit Hilfe eines in den Knochen eingebrachten Marknagels 91. Der Marknagel 91 weist zumindest eine Ausnehmung 92 auf, durch die zumindest ein Halteelement 4 einer Knochenschraube 1 hindurchragt. In dem dargestellten Ausführungsbeispiel sind es zwei Knochenschrauben 1 mit unterschiedlichen Durchmessern. Eine Verschlussschraube 93 verschließt den Marknagel nach außen, und eine Verriegelungsschraube verhindert eine Bewegung in Längsrichtung.

Eine zweite Ausführungsform der in Fig. 1 dargestellten modularen Knochenschraube 1 unterscheidet sich von der ersten Ausführungsform darin, dass anstelle der Spitze 3 eine kanulierte Spitze 5 und anstelle des Halteelements 4 ein kanuliertes Halteelement 6 vorgesehen ist.

Die kanulierte Spitze 5 unterscheidet sich von der Spitze 3 dadurch, dass zusätzlich eine koaxiale Bohrung 53 zum Hindurchleiten eines Wirkstoffs vorgesehen ist.

Das kanulierte Halteelement 6 unterscheidet sich von dem Halteelement 4 dadurch, dass zusätzlich eine koaxiale Bohrung 65 in vorgesehen ist und dass der stabförmige Abschnitt 63 nicht wie der stabförmige Abschnitt 43 bei der ersten Ausführungsform über seine gesamte Länge mit einem metrischen Gewinde versehen ist, sondern nur in einem Teilabschnitt 66.

Alle anderen Merkmale stimmen mit der ersten Ausführungsform überein, und auch der Einsatz erfolgt in der gleichen Weise.

Diese Ausführungsform hat den Vorteil, dass sich auch ohne Herausschrauben des Halteelements und auch im Nachhinein z.B. mittels einer Spritze ein medikamentöser Wirkstoff präzise an die gewünschte Stelle einbringen lässt.

Eine dritte Ausführungsform der in Fig. 1 dargestellten modularen Knochenschraube 1 unterscheidet sich von der ersten und zweiten Ausführungsform darin, dass anstelle der Spitze 3 eine selbstschneidende Spitze 7 vorgesehen ist. Alle anderen Merkmale stimmen mit der ersten bzw. zweiten Ausführungsform überein, und auch der Einsatz erfolgt in der gleichen Weise.

Durch die selbstschneidende Spitze wird das Einbringen der Knochenschraube erleichtert.

Eine in Fig. 5 dargestellte vierte Ausführungsform unterscheidet sich von der in Fig. 1 und 2 dargestellten ersten bis dritten Ausführungsform in der Form des Gewindeabschnitts.

Die Knochenschraube 10 nach der vierten Ausführungsform beinhaltet einen Schraubenabschnitt 11 und eine Spitze. Der Schraubenabschnitt 11 ist rohrförmig ausgebildet und besteht aus einem Gewindeabschnitt 12 und einem Abschnitt 13. Als Spitze kann jede der in der ersten bis dritten Ausführungsform beschriebenen Spitzen 3, 5 und 7 verwendet werden.

Der Gewindeabschnitt 12 weist wie der bei der ersten Ausführungsform beschriebene Gewindeabschnitt 2 in seiner Wandung eine Vielzahl von Ausnehmungen 21 und auf seiner Außenwandung ein Knochengewinde 22 auf.

Der Abschnitt 13 ist dagegen knochengewindefrei ausgebildet. In dem dargestellten Ausführungsbeispiel sind in dem knochengewindefreien Abschnitt 13 in der Wandung keine Ausnehmungen ausgebildet. Es können jedoch auch hier Ausnehmungen vorgesehen sein.

Auf der Innenwandung weist der rohrförmige Schraubenabschnitt 11 an seinen beiden Enden je ein metrisches Innengewinde 14, 15 auf .

Die axiale Länge des knochengewindefreien Abschnitts 13 ist in Abhängigkeit von dem Einsatzort der Knochenschraube so gewählt, dass der Gewindeabschnitt 12 an die erforderliche Stelle in dem Knochen versenkbar ist. Sie kann insbesondere auch größer sein als die axiale Länge des Gewindeabschnitts 12.

Der rohrförmige Schraubenabschnitt 11 ist vorzugsweise aus Titan geformt. Es kann jedoch auch ein anderes biokompatibles Material verwendet werden.

Auch der Einsatz der vierten Ausführungsform erfolgt in der gleichen Weise wie bei der ersten bis dritten Ausführungsform. Zum Eindrehen der Knochenschraube 10 kann auf das Innengewinde 15 ein Halteelement 4, 6 aufgeschraubt werden, das später wieder entfernt wird oder verbleibt, oder ein Kopf mit einem Angriffselement für ein Einschraubwerkzeug. Das freie Ende des Abschnitts 13 ohne Knochengewinde kann auch selber ein Angriffselement für ein Einschraubwerkzeug aufweisen wie z.B. einen Kreuzschlitz.

Die beschriebenen Ausführungsformen stellen lediglich Beispiele für die Ausführung der Erfindung dar. Gewindeabschnitt, Spitze und Halteelement können nicht nur wie in den Ausführungsformen beschrieben miteinander kombiniert werden, sondern in beliebiger Weise. Dieser modulare Aufbau ermöglich eine Anpassung an die unterschiedlichsten Anforderungen.

Der Anschlag zum Begrenzen des Einschraubwegs des Halteelements 4, 6 in den Gewindeabschnitt 2 kann statt an dem Halteelement 4, 6 auch in dem rohrförmigen Gewindeabschnitt 2 vorgesehen sein, z.B. am Ende des Innengewindes 24.

Das Halteelement 4, 6 kann so ausgebildet sein, dass der stabförmige Abschnitt 43, 63 wie bei der ersten Ausführungsform über seine ganze Länge ein Außengewinde aufweist oder wie bei der zweiten Ausführungsform nur in einem Teilabschnitt. Der stabförmige Abschnitt 43, 63 kann aber auch gänzlich ohne Außengewinde ausgebildet sein. Der Durchmesser des stabförmigen Abschnitts 43, 63 kann wie in Fig. 1 und 2 dargestellt derselbe sein wie der des ersten Abschnitts 41, 61 des Halteelements 4, 6, er kann aber auch einen anderen, vorzugsweise kleineren Durchmesser aufweisen.

Auch der Halteabschnitt 14 nach der vierten Ausführungsform kann über seine ganze Länge ein Außengewinde aufweisen oder wie bei der zweiten Ausführungsform nur in einem Teilabschnitt.

Anstelle des Außensechskantabschnitts 44, 64 kann auch ein beliebiges anderes Angriffselement für ein Einschraubwerkzeug vorgesehen sein. So kann das freie Ende des Halteelement 4, 6 z.B. auch eine Ausnehmung für einen Inbusschlüssel aufweisen. Das Halteelement 4, 6 kann auch ohne Angriffselement 44, 64 für ein Einschraubwerkzeug ausgebildet sein. Das Einschrauben der Knochenschraube erfolgt dann mit einem geeigneten Werkzeug, das an dem stabförmigen Abschnitt 43 angreift.

Die Gewindeabschnitte 23, 24, 41, 43, 61, 66, 14 und 15 sind in den Ausführungsformen als metrisch beschrieben. Alternativ dazu können die Gewinde auch zöllig oder in einer anderen geeigneten Form ausgebildet sein.

Anstelle der Innengewinde 23 und 24 bzw. 14 und 15 kann auch ein einziges Innengewinde ausgebildet sein, das sich über die gesamte Länge des Gewindeabschnitts 2 bzw. des Schraubenabschnitts 11 erstreckt. Das hat den Vorteil, dass das rohrförmige Gewindeabschnitt 2 auf jede beliebige Länge abgelängt werden kann, so dass Schrauben gewünschter Länge herstellbar sind und dadurch die Lagerhaltung wesentlich verringert werden kann.

Alternativ zu den rautenförmigen Ausnehmungen 21 können auch andere Öffnungsformen vorgesehen sein, insbesondere runde Öffnungen.

Die Spitze 3, 5, 7 kann nicht nur durch Einschrauben mit dem Gewindeabschnitt 2 bzw. 12 verbunden werden, sondern das erste Ende des Gewindeabschnitts 2 bzw. 12 und der Schaft 32, 52, 72 der Spitze 3, 5, 7 können ohne die jeweiligen Gewinde ausgebildet und in ihren Abmessungen so bestimmt sein, dass die Spitze 3, 5, 7 im Passsitz mit dem Gewindeabschnitt 2 bzw. 12 fest verbunden ist. Alternativ dazu kann die Spitze 3, 5, 7 auf eine beliebige andere Weise mit dem Gewindeabschnitt 2 bzw. 12 fest verbunden sein. Spitze und Gewindeabschnitt können auch einstückig ausgeführt sein.

Auch das Halteelement 4, 6 kann im Passsitz oder auf eine beliebige andere Weise mit dem Gewindeabschnitt 2 fest verbunden sein.

Alternativ zu der in Fig. 3 a) bis c) gezeigten Vorgehensweise kann der rohrförmige Gewindeabschnitt 2 vor dem Aufschrauben des Halteelements z.B. mit Knochenmaterial gefüllt werden. Anschließend wird die Schraube dann versenkt.

Die Schraube kann auch in dem versenkten Zustand verbleiben, ohne dass sie über das Halteelement mit einer Platte verbunden wird.

Die mit Bezug auf Fig. 3 beschriebene Kombination von Zugentlastung und Fusion kann natürlich nicht nur bei dem als Beispiel erwähnten Oberschenkelknochen durchgeführt werden, sondern bei beliebigen anderen Knochen, z.B. Tibia.

Die Knochenschraube 1, 10 ist nicht nur, wie in den Ausführungsbeispielen dargestellt, für das Einbringen in Röhrenknochen geeignet. Sie kann z.B. auch in Wirbeln oder anderen Knochen eingesetzt werden.

Das Halteelement 4, 6 kann nicht nur in Kombination mit dem rohrförmigen Gewindeabschnitt 2 verwendet werden, sondern auch mit einer beliebigen anderen Form von Knochenschrauben, die z.B. massiv oder kanuliert ausgebildet sein kann. Die Verbindung mit der Knochenschraube erfolgt in gleicher Weise wie die oben beschriebene Verbindung mit dem Gewindeabschnitt 2.

## Patentansprüche

1. Knochenschraube bestehend aus einem rohrförmig ausgebildeten Abschnitt (2) mit einer Spitze (3) an seinem ersten Ende und einem diesem gegenüberliegenden zweiten Ende, wobei der rohrförmige Abschnitt (2) auf seiner Außenwandung ein Knochengewinde (22) aufweist und die Wandung des rohrförmigen Abschnitts (2) eine Mehrzahl von Ausnehmungen (21) aufweist; und
einem Halteelement (4, 6) mit
einem ersten Abschnitt (41, 61) zum Verbinden mit dem zweiten Ende des rohrförmigen Abschnitts (2) und
einem stabförmigen zweiten Abschnitt (43, 63), der in dem eingesetzten Zustand des Halteelements (4, 6) über das zweite Ende des rohrförmigen Abschnitts (2) hinausragt,
wobei ein Anschlag vorgesehen ist zum Begrenzen des Einsetzwegs des Halteelements (4, 6) in den rohrförmigen Abschnitt (2), und der Außendurchmesser des Halteelements (4, 6) nicht größer als der Außendurchmesser des rohrförmigen Abschnitts ist, derart, dass die Knochenschraube vollständig in der beim Einschrauben in den Knochen geschaffenen Öffnung versenkbar ist, und das Halteelement wieder herausführbar ist.

2. Knochenschraube nach Anspruch 1, **dadurch gekennzeichnet, dass**
der rohrförmig ausgebildete Abschnitt (2) an seinem zweiten Ende einen Innengewindeabschnitt (24) aufweist und
der erste Abschnitt (41, 61) des Halteelements (4, 6) ein Außengewinde aufweist zum Einschrauben in den Innengewindeabschnitt (24).

3. Knochenschraube nach Anspruch 1, **dadurch gekennzeichnet, dass** die Verbindung zwischen dem Halteelement (4, 6) und dem rohrförmigen Abschnitt (2) in Form eines Presssitzes erfolgt.

4. Knochenschraube nach einem der Ansprüche 1 bis 3, **dadurch gekennzeichnet, dass** das Halteelement (4, 6) an seinem freien Ende ein Element (44, 64) aufweist zum Ein- oder Angreifen mit einem Einschraubwerkzeug.

5. Knochenschraube nach einem der Ansprüche 1 bis 4, **dadurch gekennzeichnet, dass** der Anschlag in Form einer Schulter (42, 62) zwischen dem ersten Abschnitt (41, 61) und dem zweiten Abschnitt (43, 63) des Halteelements vorgesehen ist.

6. Knochenschraube nach einem der Ansprüche 1 bis 4, **dadurch gekennzeichnet, dass** der Anschlag in dem rohrförmigen Abschnitt (2) der Knochenschraube (1) vorgesehen ist.

7. Knochenschraube nach einem der Ansprüche 1 bis 6, **dadurch gekennzeichnet, dass** der stabförmige Abschnitt (43, 63) des Halteelements (4, 6) ein Außengewinde aufweist zum Aufschrauben einer Mutter (83) von dem freien Ende des Halteelements (4, 6) her.

8. Knochenschraube nach einem der Ansprüche 1 bis 7, **dadurch gekennzeichnet, dass**
der rohrförmig ausgebildete Abschnitt (2) an seinem ersten Ende einen Innengewindeabschnitt (23) aufweist oder der Innengewindeabschnitt sich über die gesamte Länge des rohrförmig ausgebildeten Abschnitts (2) erstreckt und
die Spitze (3, 5, 7) an dem ersten Ende des rohrförmigen Abschnitts (2) in diesen Innengewindeabschnitt (23) einschraubbar ist.

9. Knochenschraube nach einem der Ansprüche 2, 4 bis 8, **dadurch gekennzeichnet, dass** der Innengewindeabschnitt (23, 24) als metrisches oder zölliges Gewinde ausgebildet ist.

10. Knochenschraube nach einem der Ansprüche 1 bis 9, **dadurch gekennzeichnet, dass** die Spitze (7) als selbstschneidende Spitze ausgebildet ist.

11. Knochenschraube nach einem der Ansprüche 1 bis 10, **dadurch gekennzeichnet, dass** die Spitze (5) kanuliert ausgebildet ist.

12. Knochenschraube nach einem der Ansprüche 1 bis 11, **dadurch gekennzeichnet, dass** das Halteelement (6) kanuliert ausgebildet ist.

13. Knochenfixationseinrichtung (80) mit
zumindest einer Knochenschraube nach einem der Ansprüche 1 bis 12,
einer Platte (81) zum Fixieren eines Knochens mit einer Ausnehmung (82), durch die das freie Ende des Stabelements (4, 6) hindurchführbar ist, und
einer Mutter (83) zum Aufschrauben auf das Halteelement (4, 6) von seinem freien Ende her und zum Fixieren der Platte (81).

14. Knochenfixationseinrichtung (90) mit
zumindest einer Knochenschraube nach einem der Ansprüche 1 bis 12, und
einem Marknagel (91) zum Fixieren eines Knochens mit einer Ausnehmung (92), durch die das freie Ende des Stabelements (4, 6) hindurchführbar ist.

## Claims

1. Bone screw consisting of a tubular formed section (2) having a tip (3) at its first end and a second end opposite to the first end, wherein the tubular section (2) comprises a bone thread (22) at its outer wall and wherein the wall of the tubular section (2) comprises a plurality of recesses (21); and
a holding element (4, 6) having
a first section (41, 61) for being connected to the second end of the tubular section (2) and
a rod-shaped second section (43, 63), which protrudes over the second end of the tubular section (2) in the inserted state of the holding element (4, 6),
wherein a stop is provided for limiting the insertion distance of the holding element (4, 6) in the tubular section (2) and the outer diameter of the holding element (4, 6) is not larger than the outer diameter of the tubular section, such that the bone screw is completely immersible in the opening which is created when screwing into the bone, and wherein the holding element is guidable out.

2. Bone screw of claim 1, **characterized in that** the tubular formed section (2) comprises an inner thread section (24) at its second end and
the first section (41, 61) of the holding element (4, 6) comprises an outer thread for screwing-in into the inner thread section (24).

3. Bone screw of claim 1, **characterized in that** the connection between the holding element (4, 6) and the tubular section (2) is effected in form of a press-fit.

4. Bone screw of one of the claims 1 to 3, **characterized in that** the holding element (4, 6) comprises an element (44, 46) at its free end for engaging or embracing with a screwing-in tool.

5. Bone screw of one of the claims 1 to 4, **characterized in that** the stop is provided in form of a shoulder (42, 62) between the first section (41, 61) and the second section (43, 63) of the holding element.

6. Bone screw of one of the claims 1 to 4, **characterized in that** the stop is provided in the tubular section (2) of the bone screw (1).

7. Bone screw of one of the claims 1 to 6, **characterized in that** the rod-shaped section (43, 63) of the holding element (4, 6) comprises an outer thread for screwing-onto a nut (83) from the free end of the holding element (4, 6).

8. Bone screw of one of the claims 1 to 7, **characterized in that** the tubular-shaped section (2) comprises an inner thread section (23) at its first end or that the inner thread section extends over the complete length of the tubular-shaped section (2) and that the tip (3, 5, 7) is screwable into this inner thread section (23) at the first end of the tubular section (2).

9. Bone screw of one of the claims 2,4 to 8, **characterized in that** the inner thread section (23, 24) is formed as metric or imperial thread.

10. Bone screw of one of the claims 1 to 9, **characterized in that** the tip (7) is formed as self-cutting tip.

11. Bone screw of one of the claims 1 to 10, **characterized in that** the tip (5) is formed canulated.

12. Bone screw of one of the claims 1 to 11, **characterized in that** the holding element (6) is formed canulated.

13. Bone fixation device (80) having at least one bone screw according to the claims 1 to 12,
a plate (81) for fixing a bone with a recess (82), through which the free end of the rod element (4, 6) is guidable through, and
a nut (83) for screwing onto the holding element (4, 6) from its free end and for fixing the plate (81).

14. Bone fixation device (90) having at least one bone screw according to one of the claims 1 to 12 and
an intramedullary nail (91) for fixing a bone with a recess (92) through which the free end of the rod element (4, 6) is guidable through.

## Revendications

1. Vis à os, constituée d'un tronçon (2) tubulaire avec une pointe (3) à sa première extrémité et une deuxième extrémité opposée à celle-ci, le tronçon (2) tubulaire présentant, sur sa paroi extérieure, un filetage à os (22) et la paroi du tronçon (2) tubulaire présentant une pluralité d'évidements (21) ; et
d'un élément de maintien (4, 6) avec un premier tronçon (41, 61) à raccorder à la deuxième extrémité du tronçon (2) tubulaire et
un deuxième tronçon (43, 63) en forme de tige, qui ressort de l'extrémité du tronçon (2) tubulaire en état de montage de l'élément de maintien (4, 6),
une butée étant prévue pour limiter la course d'insertion de l'élément de maintien (4, 6) dans le tronçon (2) tubulaire, et le diamètre extérieur de l'élément de maintien (4, 6) n'étant pas supérieur au diamètre extérieur du tronçon tubulaire, si bien que la vis à os peut être intégralement logée dans l'ouverture créée par vissage dans l'os, et que l'élément de maintien en est amovible.

2. Vis à os selon la revendication 1, **caractérisée en ce que**
le tronçon (2) tubulaire présente à sa deuxième extrémité un tronçon à filetage intérieur (24), et
le premier tronçon (41, 61) de l'élément de maintien (4, 6) présente un filetage extérieur pour être vissé dans le tronçon à filetage intérieur (24).

3. Vis à os selon la revendication 1, **caractérisée en ce que** la connexion entre l'élément de maintien (4, 6) et le tronçon (2) tubulaire est réalisée sous la forme d'un ajustage serré.

4. Vis à os selon l'une des revendications 1 à 3, **caractérisée en ce que** l'élément de maintien (4, 6) présente à son extrémité libre un élément (44, 64) pour la prise extérieure ou intérieure d'un outil à visser.

5. Vis à os selon l'une des revendications 1 à 4, **caractérisée en ce que** la butée est prévue sous la forme d'un épaulement (42, 62) entre le premier tronçon (41, 61) et le deuxième tronçon (43, 63) de l'élément de maintien.

6. Vis à os selon l'une des revendications 1 à 4, **caractérisée en ce que** la butée est prévue dans le tronçon (2) tubulaire de la vis à os (1).

7. Vis à os selon l'une des revendications 1 à 6, **caractérisée en ce que** le tronçon (43, 63) en forme de tige de l'élément de maintien (4, 6) présente un filetage extérieur pour le vissage d'un écrou (83) depuis l'extrémité libre de l'élément de maintien (4, 6).

8. Vis à os selon l'une des revendications 1 à 7, **caractérisée en ce que**
le tronçon (2) tubulaire présente à sa première extrémité un tronçon à filetage intérieur (23), ou le tronçon à filetage intérieur s'étend sur toute la longueur du tronçon (2) tubulaire, et
la pointe (3, 5, 7) est vissable dans ledit tronçon à filetage intérieur (23) à la première extrémité du tronçon (2) tubulaire.

9. Vis à os selon l'une des revendications 2,4 à 8, **caractérisée en ce que** le tronçon à filetage intérieur (23, 24) est réalisé avec un filetage métrique ou un filetage au pouce.

10. Vis à os selon l'une des revendications 1 à 9, **caractérisée en ce que** la pointe (7) est réalisée comme pointe autoperforante.

11. Vis à os selon l'une des revendications 1 à 10, **caractérisée en ce que** la pointe (5) est canulée.

12. Vis à os selon l'une des revendications 1 à 11, **caractérisée en ce que** l'élément de maintien (6) est canulé.

13. Dispositif de fixation osseuse (80), avec
au moins une vis à os selon l'une des revendications 1 à 12,
une plaque (81) pour la fixation d'un os, avec un évidement (82) que peut traverser l'extrémité libre de l'élément de maintien (4, 6), et
un écrou (83) à visser sur l'élément de maintien (4, 6) depuis l'extrémité libre de celui-ci et destiné à fixer la plaque (81).

14. Dispositif de fixation osseuse (90), avec
au moins une vis à os selon l'une des revendications 1 à 12, et
un clou à moelle (91) pour la fixation d'un os, avec un évidement (92) où peut être introduite l'extrémité libre de l'élément de maintien (4, 6).
